(19)

Europäisches
Patentamt
European
Patent Office
Office européen
des brevets

(11) **EP 4 311 542 A1**

(12) **EUROPEAN PATENT APPLICATION**

(43) Date of publication:
**31.01.2024 Bulletin 2024/05**

(21) Application number: **22306124.3**

(22) Date of filing: **27.07.2022**

(51) International Patent Classification (IPC):
***A61K 9/24*** (2006.01)

(52) Cooperative Patent Classification (CPC):
**A61K 9/0065; A61K 9/209**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **ROQUETTE FRERES
62136 Lestrem (FR)**

(72) Inventor: **The designation of the inventor has not
yet been filed**

(74) Representative: **Plasseraud IP
66, rue de la Chaussée d'Antin
75440 Paris Cedex 09 (FR)**

(54) **FLOATING TAB-IN-TAB DOSAGE FORMS**

(57)     The invention relates to new gastroretentive tablets that are floating tab-in-tab dosage forms. The invention also relates to a process for obtaining thereof, as well as to the use thereof for treating diseases or conditions in which such gastroretentive tablets are useful.

EP 4 311 542 A1

**Description**

**Technical Field**

The invention pertains to the field of gastro-retentive tablets, more specifically to the field of floating tablets.

**Background Art**

Oral drug delivery systems have dominated other drug delivery systems for human administration due to their various advantages including ease of administration, flexibility in formulation, cost-effectiveness, easy storage and transport, and high patient compliance. However, oral drug delivery systems face challenges such as low bioavailability due to the heterogeneity of the gastrointestinal system, pH of the gastro-intestinal environment, gastric retention time of the dosage form, surface area, and enzymatic activity. Conventional drug delivery systems may not overcome the issues imposed by the gastrointestinal tract (GIT) such as incomplete absorption of drugs, decrease in dose effectiveness, and frequent dose requirement. Therefore, the failure of conventional drug delivery systems to retain drugs in the stomach may lead to the development of gastro-retentive drug delivery system (GRDDS). These systems offer several benefits such as prolonged gastric residence time of dosage forms in the stomach up to several hours, increased therapeutic efficacy of drugs by improving drug absorption, and suitability for targeted delivery in the stomach. In addition, GRDDS can enhance the controlled delivery of drugs by continuously releasing the drug for an extended period at the desired rate and to the desired absorption site until the drug is completely released from the dosage form.

In the recent decades, several technologies have evolved showing different mechanisms for retaining the drug in the GIT for longer duration with increased bioavailability. Floatable, mucoadhesive, swellable, magnetic, nanofibrous, high-density, and expandable systems have been investigated extensively as the potential gastro-retentive strategies.

Floating systems include non-effervescent and effervescent systems. In the gas-generating floating system, effervescent agents such as sodium bicarbonate, calcium carbonate, tartaric acid, and citric acid are used in combination with hydrophilic polymers. When this system comes into contact with gastric fluid, $CO_2$ is released due to the reaction of the effervescent agent with gastric fluid. The released $CO_2$ gas is entrapped in the hydrocolloid matrix, which provides the tablet buoyancy and influences the drug release properties.

The problem with conventional floating systems is that drug release follows a logarithmic curve, meaning that the bioavailability of the drug varies over the time, resulting in safety and efficacy challenges to deliver drugs within the therapeutic window.

Interestingly, patent application CN104688703 A offers a floating GRDDS in which linear release of the drug is obtained. This GRDDS is characterized by the fact that it is in the form of a tab-in-tab dosage form: a small tablet (core) is embedded in a bigger tablet (shell). The core contains ofloxacin and a hydrophilic gel material, and the shell layer contains ofloxacin, a hydrophilic gel material, a disintegration inhibitor and a gas generating agent. Linear release of ofloxacin is achieved for instance by using the shell and core formulations of Example 1 or 2. In these formulations, hydroxypropyl cellulose is used as a hydrophilic gel material, sodium alginate is used as a disintegration inhibitor, and sodium bicarbonate is used as a gas generating agent.

CN104688703 A offers an original approach for achieving linear release of the drug. Indeed, the tab-in-tab technology had never been used in the field of floating tablets. Instead, the tab-in-tab technology has been used for a completely different purpose, that is to deliver two different drugs with two different kinetics: the shell quickly releases a first drug, while the core slowly releases second drug. In CN104688703 A, one same drug is present both in the core and in the shell, and the shell is effervescent.

Unfortunately, the floating tab-in-tab dosage forms of CN104688703 A don't systematically exhibit a linear release of the drug, as evidenced by Example 3.

Most importantly, the tab-in-tab dosage forms as those of CN104688703 A lack gel strength.

However, a dosage form with sustained high gel strength is important in order to resist gastric churning forces. In the case of high drug load floating formulations, the risk of weak gel strength is greater due to the limited excipient working space and presence of gas bubbles further weaken the hydrated mass.

This problem of gel strength has not been well studied. There was thus an unsatisfied need for a floating dosage form with improved gel strength, while being able to achieve linear release of a drug.

**Technical Problem**

It was thus an objective of the invention to provide a gastro-retentive floating tablet with improved properties, especially as compared to CN104688703 A.

It was another objective of the invention to provide a gastro-retentive floating tablet with improved bioavailability, especially capable of providing linear release of a drug.

It was another objective of the invention to provide a gastro-retentive floating tablet with satisfying gel strength.

It was another objective of the invention to provide a gastro-retentive floating tablet with satisfying floating lag time and/or satisfying tensile strength.

It was another objective of the present invention to provide a gastro-retentive floating tablet that possess the typical other properties required for this type of dosage forms, notably in terms of safety and stability.

It was another objective of the present invention to provide a gastro-retentive floating tablet which can be easily prepared, for instance using directly compressible and free-flowing materials.

It was another objective of the present invention to provide a gastro-retentive floating tablet using materials allowing correct filling of the matrices, i.e. uniform and reproducible filling with a precise amount of powder, and/or flowing correctly in the equipment used in direct compression, and/or which are chemically and physically stable, and/or which are sufficiently cohesive to allow them to be transported or to allow mixtures to be prepared, and/or that do not hinder the bioavailability of the active ingredient, and/or allowing homogeneous mixing of the ingredients of the composition, and/or having a good absorption capacity, and/or allowing the production of tablets with an acceptable texture and an acceptable taste, and/or generating packaging and transportation costs which adhere to commercial standards.

**Presentation of the invention**

The inventors solved this technical problem by providing a floating tab-in-tab dosage form comprising an active ingredient, said tab-in-tab dosage form comprising a core (a) and a shell (b), wherein:

the core (a) comprises a first portion of said active ingredient;
the shell (b) comprises:

- a second portion of said active ingredient; and,
- a release retarding agent; and,
- a gelling agent capable of being ionically cross-linked in the presence of divalent cations; and,
- an acid-soluble divalent cation; and,
- a bicarbonate salt.

Preferably, the acid-soluble divalent cation is a carbonate. In that case, it can advantageously also act as a gas-generating agent, together with the bicarbonate.

The tab-in-tab dosage forms according to the disclosure allow: to achieve linear release of a drug, while exhibiting satisfying tensile strength and floating lag time (Examples 2.1, 2.2). Furthermore, excellent gel strength could be obtained (Example 2.3), especially when using a release retarding agent / gelling agent weight ratio lower than 3.0 in the shell, in particular lower than 2.5.

When the bicarbonate salt was omitted, the tablets were not able to float properly (Example 2.2). When the bicarbonate salt or the divalent cation were omitted (like in CN104688703 A previously mentioned), the gel strength was not satisfying (Example 2.3).

Furthermore, by using the tab-in-tab dosage forms according to the disclosure, it is possible to obtain tablets with high drug load, for instance of about 50% w/w. This is particularly remarkable, considering that the active ingredient used in the Example below is very soluble. Indeed, soluble active ingredients tend to weaken the gel of the solid dosage form. However, the solid dosage forms according to the disclosure exhibit good gel strength, despite the high amounts of soluble drugs used.

**Brief description of the invention**

The invention first relates to a floating tab-in-tab dosage form comprising an active ingredient, said tab-in-tab dosage form comprising a core (a) and a shell (b), wherein:

the core (a) comprises a first portion of said active ingredient;
the shell (b) comprises:

- a second portion of said active ingredient; and,
- a release retarding agent; and,
- a gelling agent capable of being ionically cross-linked in the presence of divalent cations; and,
- an acid-soluble divalent cation; and,
- a bicarbonate salt.

Preferably, the acid-soluble divalent cation is selected from carbonate salts, preferably from calcium carbonate, magnesium carbonate, zinc carbonate, calcium chloride, or from a mixture thereof.

Preferably, the bicarbonate salt is selected from ammonium bicarbonate, lithium bicarbonate, potassium bicarbonate, sodium bicarbonate, or from a mixture thereof.

Preferably, the release retarding agent is selected from hydroxypropyl methylcellulose (HPMC), hydroxyethylcellulose (HEC), hydroxypropyl cellulose (HPC), native or chemically modified pregelatinized starches, guar gum, gellan gum, xanthan gum, their derivatives, or from a mixture thereof.

Preferably, the gelling agent capable of being ionically cross-linked in the presence of divalent cations is selected from alginate, pectinate, iota-carrageenan, gellan gum, carboxymethylcellulose, or from a mixture thereof.

Preferably, the weight ratio of release retarding agent / gelling agent of the shell is equal to or higher than 0.5, preferably equal to or higher than 1.0, preferably equal to or higher than 1.5, preferably higher than 1.5, preferably equal to or higher than 2.0.

Preferably, the weight ratio of release retarding agent / gelling agent of the shell is equal to or lower than 5.0, preferably equal to or lower than 4.5, preferably equal to or lower than 3.5, preferably equal to or lower than 3.0, preferably lower than 3.0, preferably equal to or lower than 2.5.

Preferably, the amount of said active ingredient is equal to or higher than 10% by weight with respect to the total weight of the tab-in-tab dosage form, preferably equal to or higher than 20%, preferably equal to or higher than 30%, preferably equal to or higher than 40%, preferably equal to or higher than 45%.

Preferably, the weight ratio between the first and second portion of said active ingredient is equal to or higher than 0.2, preferably equal to or higher than 0.4, preferably equal to or higher than 0.6, preferably equal to or higher than 0.7.

Preferably, the weight ratio between the first and second portion of said active ingredient is equal to or lower than 3.0, preferably equal to or lower than 2.5, preferably equal to or lower than 2.0, preferably equal to or lower than 1.7, preferably equal to or lower than 1.5, preferably lower than 1.5, preferably equal to or lower than 1.4, preferably equal to or lower than 1.3, preferably equal to or lower than 1.2, preferably equal to or lower than 1.1.

The invention also relates to a process for the manufacture of a floating tab-in-tab dosage form comprising at an active ingredient, said process comprising:

- a step of obtaining a first tablet comprising a first portion of said active ingredient; and,
- a step of including said first tablet into a second tablet, said second tablet comprising a second portion of said active ingredient, a release retarding agent, a gelling agent capable of being ionically cross-linked in the presence of divalent cations, an acid-soluble divalent cation and a bicarbonate salt.

The invention also relates to the use of the floating tab-in-tab dosage form according to the invention, or obtainable by the process for the manufacture of a floating tab-in-tab dosage according to the invention, for use as a medicine.

**Brief Description of Drawings**

**Fig. 1**

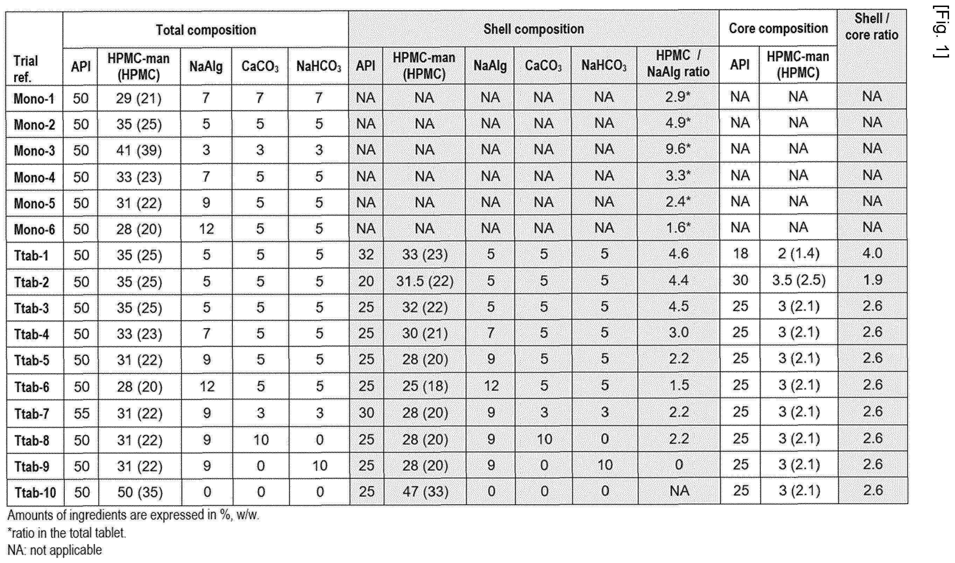

[Fig. 1] is a table showing the formulations of the dosage forms (monolithic or tab-in-tab dosage forms) prepared and evaluated in the Example section.

**Fig. 2**

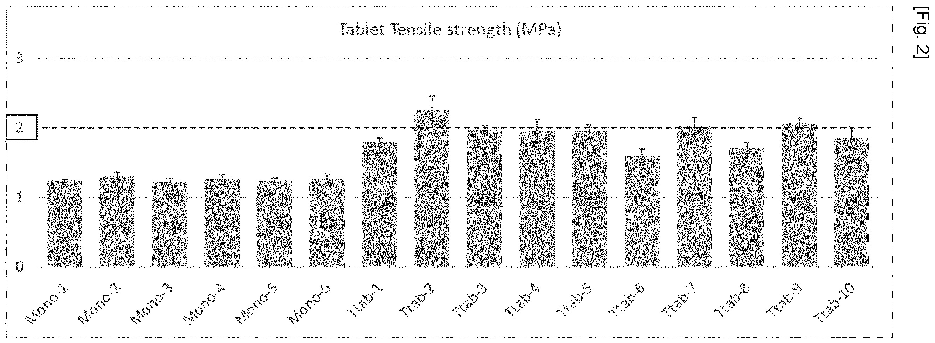

[Fig. 2] is a bar chart showing the tensile strength of various tablets (monolithic or tab-in-tab dosage forms).

**Fig. 3**

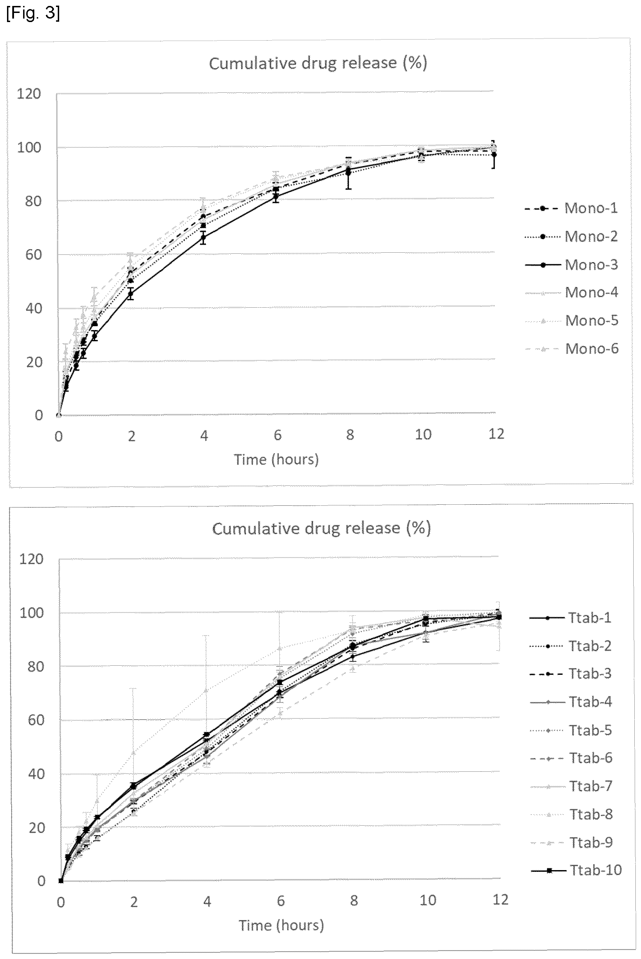

[Fig. 3] is a graph showing the drug release profile of various tablets (monolithic dosage forms on the top graph; tab-in-tab dosage forms on the bottom graph).

**Fig. 4**

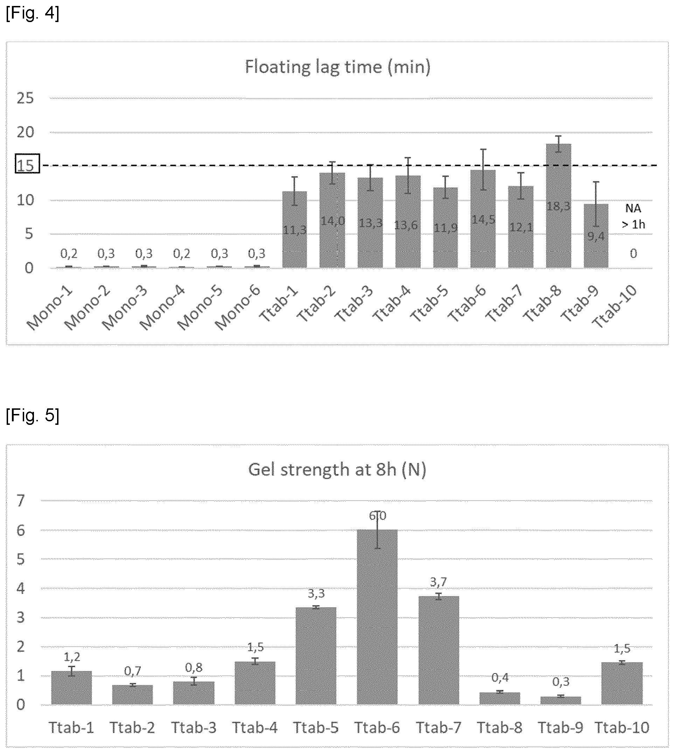

[Fig. 4] is a bar chart showing the floating lag time of tab-in-tab dosage forms (comparative or according to the disclosure).

**Fig. 5**
[Fig. 5] is a bar chart showing the gel strength of tab-in-tab dosage forms (comparative or according to the disclosure).

**Description of Embodiments**

The invention first relates to a floating tab-in-tab dosage form comprising an active ingredient, said tab-in-tab dosage form comprising a core (a) and a shell (b), wherein:

the core (a) comprises a first portion of said active ingredient;
the shell (b) comprises:

-    a second portion of said active ingredient; and,
-    a release retarding agent; and,
-    a gelling agent capable of being ionically cross-linked in the presence of divalent cations; and,
-    an acid-soluble divalent cation; and,
-    a bicarbonate salt.

The dosage form of the invention is a "tab-in-tab" dosage form. It is commonly understood that this refers to a system in which the entire surface of an inner core is surrounded by a shell. It is sometimes also referred as "compression coated tablets". In other words, it is a tablet embedded in another a tablet.

The dosage form according to the disclosure is a floating tablet, in particular, an effervescent floating tablet, especially a gas-generating effervescent floating tablet. It is a gastro-retentive tablet and is intended to be delivered orally.

The shell of the tab-in-tab dosage form according to the disclosure comprises an acid-soluble divalent cation.

Preferably, the acid-soluble divalent cation is selected from carbonate salts, preferably from calcium carbonate, magnesium carbonate, zinc carbonate, calcium chloride, or from a mixture thereof. It is preferably selected from carbonates. It is still preferably calcium carbonate ($CaCO_3$).

Preferably, the amount of acid-soluble divalent cation of the shell is equal to or higher than 1% by weight with respect to the total weight of the tab-in-tab dosage form, preferably equal to or higher than 2%. It is preferably equal to or lower than 10%, preferably equal to or lower than 9%, preferably equal to or lower than 8%, preferably equal to or lower than 7%, preferably equal to or lower than 6%. It is for example equal to about 3 to 5%.

It is understood that the weight of divalent cation also includes the counterion. For instance, for $CaCO_3$, X% of divalent cation means X% of $CaCO_3$, and not X% of $Ca^{2+}$.

The shell of the tab-in-tab dosage form according to the disclosure further comprises a bicarbonate salt.

The bicarbonate salt may be selected for example from ammonium bicarbonate, lithium bicarbonate, potassium bicarbonate, sodium bicarbonate, or from a mixture thereof. It is still preferably sodium bicarbonate.

Preferably, the amount of bicarbonate salt of the shell is equal to or higher than 1% by weight with respect to the total weight of the tab-in-tab dosage form, preferably equal to or higher than 2%. It is preferably equal to or lower than 10%, preferably equal to or lower than 9%, preferably equal to or lower than 8%, preferably equal to or lower than 7%, preferably equal to or lower than 6%. It is for example equal to about 3 to 5%.

The shell of the tab-in-tab dosage form according to the disclosure further comprises a release retarding agent.

The release regarding agent typically is a hydrophilic polymer. It is typically capable of forming a gel.

The release retarding agent can be selected from: hydroxypropyl methylcellulose (HPMC), hydroxyethylcellulose (HEC), hydroxypropyl cellulose (HPC), native or chemically modified pregelatinized starches, guar gum, xanthan gum, their derivatives, or from a mixture thereof.

The release regarding agent according to the disclosure may be compressible or not. Preferably, the release regarding agent used for preparing the tab-in-tab dosage form is directly compressible, meaning that there is no need for it to be granulated before tableting. It can for instance be selected from the compressible forms of HPMC (e.g. METHOCEL™ DC2, Benecel™ DC, RetaLac®), including the compressible forms obtained by co-processing HPMC with a polyol for example as described in patent US 9,234,049 B2, or from a mixture thereof. It is preferably a co-processed HPMC / mannitol compound having preferably a weight ratio HPMC : mannitol of 70:30.

Preferably, the amount of release retarding agent of the shell is equal to or higher than 5% by weight with respect to the total weight of the tab-in-tab dosage form, preferably equal to or higher than 10%, preferably equal to or higher than 15%, preferably equal to or higher than 18%, preferably equal to or higher than 19%. It is preferably equal to or lower than 40%, preferably equal to or lower than 35%, preferably equal to or lower than 30%, preferably equal to or lower than 25%, preferably equal to or lower than 23%, preferably equal to or lower than 22%, preferably equal to or lower than 21%. It is for example equal to about 20%.

It is understood that if the release retarding agent is co-processed with a compound intended to make it compressible (e.g. a co-processed HPMC / mannitol compound in a weight ratio of 70/30 like used in the Examples), only the release retarding agent portion (i.e. HPMC in this co-processed HPMC / mannitol compound) is taken into consideration for the calculation of the amounts and ratios.

The shell of the dosage forms according to the disclosure further comprises a gelling agent capable of being ionically cross-linked in the presence of divalent cations. These gelling agents typically are salts of compounds bearing anionic groups.

This gelling agent can for example be selected from alginate, pectinate, iota-carrageenan, gellan gum, carboxymethylcellulose, or from a mixture thereof. It is preferably alginate, still preferably sodium alginate.

Preferably, the amount of gelling agent of the shell is equal to or higher than 1% by weight with respect to the total weight of the tab-in-tab dosage form, preferably equal to or higher than 3%, preferably equal to or higher than 5%, preferably higher than 5%, preferably equal to or higher than 7%, preferably higher than 7%, preferably equal to or higher than 8%. It is preferably equal to or lower than 20%, preferably equal to or lower than 15%, preferably equal to or lower than 12%, preferably lower than 12%, preferably equal to or lower than 11%, preferably equal to or lower than 10%. It is for example equal to about 9%.

Preferably, the weight ratio of release retarding agent / gelling agent of the shell, is equal to or higher than 0.5, preferably equal to or higher than 1.0, preferably equal to or higher than 1.5, preferably higher than 1.5, preferably equal to or higher than 2.0. It is preferably equal to or lower than 5.0, preferably equal to or lower than 4.5, preferably equal to or lower than 3.5, preferably equal to or lower than 3.0, more preferably lower than 3.0, preferably equal to or lower than 2.5. It is for example equal to about 2.0. It is preferably selected from 1.5 to 3.0, or from 1.5 to 2.5, or from 2.0 to 3.0, or from 2.0 to 2.5.

The tab-in-tab dosage form according to the disclosure comprises an active ingredient. A first portion of said active ingredient is included in its core. A second portion is included in its shell.

Preferably, the amount of said active ingredient of the tab-in-tab dosage is equal to or higher than 10% by weight with respect to the total weight of the tab-in-tab dosage form, preferably equal to or higher than 20%, preferably equal to or higher than 30%, preferably equal to or higher than 40%, preferably equal to or higher than 45%. It is in general equal to or lower than 80%, preferably equal to or lower than 70%, preferably equal to or lower than 60%, preferably equal to or lower than 55%, preferably equal to or lower than 50%. It is for example equal to about 50%.

Preferably, the amount of said first portion of said active ingredient (core portion) is equal to or higher than 5% by weight with respect to the total weight of the tab-in-tab dosage form, preferably equal to or higher than 10%, preferably equal to or higher than 15%, preferably equal to or higher than 18%, preferably higher than 18%, preferably equal to or higher than 20%. It is preferably equal to or lower than 50%, preferably equal to or lower than 40%, preferably equal to or lower than 30%, preferably lower than 30%. It is for example equal to about 25%.

Preferably, the amount of said second portion of said active ingredient (shell portion) is equal to or higher than 5% by weight with respect to the total weight of the tab-in-tab dosage form, preferably equal to or higher than 10%, preferably equal to or higher than 15%, preferably equal to or higher than 20%, preferably higher than 20%. It is preferably equal to or lower than 50%, preferably equal to or lower than 40%, preferably equal to or lower than 32%, preferably lower than 32%, preferably equal to or lower than 30%. It is for example equal to about 25 to 30%.

Preferably, in the tab-in-tab dosage form according to the disclosure, the weight ratio between the first and second portion of said active ingredient is equal to or higher than 0.2, preferably equal to or higher than 0.4, preferably equal to or higher than 0.6, preferably equal to or higher than 0.7. It is preferably equal to or lower than 3.0, preferably equal to or lower than 2.5, preferably equal to or lower than 2.0, preferably equal to or lower than 1.7, preferably equal to or lower than 1.5, more preferably lower than 1.5, preferably equal to or lower than 1.4, preferably equal to or lower than 1.3, preferably equal to or lower than 1.2, preferably equal to or lower than 1.1. It is for example equal to about 0.8 to 1.0.

The active ingredient according to the disclosure includes non-pharmaceutical and pharmaceutical agents. The expression "active" classically refers to any substance of pharmaceutical, veterinary, food, nutraceutical, or cosmetic interest, which are intended to be taken orally. Preferably, the active ingredient according to the disclosure is a pharmaceutical active ingredient. The active ingredient, in particular when it is a pharmaceutical active ingredient, may be chosen from so-called small molecules, but also from so-called "biologics", as is the case for example of active substance based on or derived from proteins, nucleic acids - such as those derived from DNA or RNA - cells or viruses.

Preferably, the active ingredient according to the disclosure is selected from active ingredients targeting the stomach e.g. anti-acids, antibiotics for *H. pylori* infection, from pharmaceutical ingredients having low absorption window, e.g. metformin, furosemide, levodopa, from pharmaceutical ingredients having poor solubility or chemical instability at high intestinal pH e.g. verapamil, diazepam, captopril, or from a mixture thereof.

The active ingredient according to the disclosure may be selected from BCS (Biopharmaceuticals Classification System) class I drugs, BCS class II drugs, BCS class III drugs or BCS class IV drugs; said classification being as defined by the US Food and Drug Administration (FDA). It is preferably selected from BCS class I or III drugs, still preferably from BCS class III drugs.

Preferably, the active ingredient of the disclosure is metformin.

The core of the tab-in-tab dosage form according to the disclosure might include ingredients other than the active ingredient according to the disclosure, as long as it does not interfere with the desired properties of the tab-in-tab dosage form, notably in terms of safety and performance. Example of such other ingredients are: additional active ingredients; fillers and/or binders, like sugars, sugar alcohols, hydroxypropyl methylcellulose, microcrystalline cellulose; colors; flavors; solubilizer, e.g. cyclodextrin compounds; lubricants, e.g. magnesium stearate.

Preferably, the core of the tab-in-tab dosage form according to the disclosure comprises a filler and/ or binder, which is preferably a direct compression excipient. Preferably, as filler and/or binder, a release retarding agent or a co-processed form thereof as defined previously is used. Preferably, the release retarding agent or co-processed form thereof is the same as the one used for the shell.

Preferably, the core of the tab-in-tab dosage form according to the disclosure is free of additional active ingredient.

Preferably, the amount of other ingredients (i.e., other than the active ingredient according to the disclosure) in the core is equal to or lower than 10% by weight with respect to the total weight of the tab-in-tab dosage form, preferably equal to or lower than 5%, preferably equal to or lower than 4%. It is in general equal to or higher than 1%, even equal to or higher than 2%. It is for example equal to about 3%.

The shell of the tab-in-tab dosage form according to the disclosure might include ingredients other than the ones described before, as long as it does not interfere with the desired properties of the tab-in-tab dosage form, notably in terms of safety and performance. Example of such other ingredients are: additional active ingredients; fillers and/or binders, like sugars, sugar alcohols, hydroxypropyl methylcellulose, microcrystalline cellulose; colors; flavors; solubilizer, e.g. cyclodextrin compounds; lubricants, e.g. magnesium stearate.

Preferably, the amount other ingredients in the shell is equal to or lower than 30% by weight with respect to the total weight of the tab-in-tab dosage form, preferably equal to or lower than 25%, preferably equal to or lower than 20%, preferably equal to or lower than 15%, preferably equal to or lower than 12%, preferably equal to or lower than 10%, preferably lower than 10%. It can be equal to 0%, even equal to or higher than 1 %, even equal to or higher than 3%, even equal to or higher than 5%, even equal to or higher than 8%, even higher than 8%. It is for example equal to about 9%. The shell can also be free of such other ingredients.

Preferably, the shell of the tab-in-tab dosage form according to the disclosure is free of additional active ingredient.

It is understood that if the release retarding agent according to the disclosure is co-processed with a compound intended to make it compressible (e.g. a co-processed HPMC / mannitol compound in a weight ratio of 70/30 like used in the Examples), the compound intended to make it compressible (i.e. mannitol in this co-processed HPMC / mannitol compound) is here considered to be an "other ingredient".

Preferably, the tab-in-tab dosage form according to the disclosure comprises:

a core (a) composed of: a first portion of said active ingredient and optionally additional ingredient(s); and,
a shell (b) composed of:

- a second portion of said active ingredient; and,
- a release retarding agent, preferably HPMC and/or co-processed HPMC and mannitol; and,
- a gelling agent capable of being ionically cross-linked in the presence of divalent cations, preferably sodium alginate; and,
- an acid-soluble divalent cation, preferably sodium carbonate; and,
- a bicarbonate salt, preferably sodium bicarbonate; and,
- optionally additional ingredient(s).

Preferably the natures and amounts of ingredients composing this tab-in-tab dosage forms are as described before.

Preferably, the tab-in-tab dosage form according to the disclosure comprises:

a core (a) comprising: a first portion of said active ingredient; and,
a shell (b) comprising:

- a second portion of said active ingredient; and,
- HPMC and/or co-processed HPMC and mannitol; and,
- sodium alginate; and,
- calcium carbonate; and,
- sodium bicarbonate.

The tab-in-tab dosage forms according to the disclosure might be further coated by any technique known to those skilled in the art, for example by film-coating or by sugar-coating.

Preferably, the composition of the core is directly compressible, that is to say that no granulation step is required in order to obtain a tablet from said composition.

Preferably, the composition of the shell is directly compressible.

Preferably, the weight ratio of shell to core of the tab-in-tab dosage form according to the disclosure is equal to or higher than 1.0, preferably equal to or higher than 1.5, preferably equal to or higher than 1.9, preferably higher than 1.9, preferably equal to or higher than 2.0, preferably equal to or higher than 2.3, preferably equal to or higher than 2.5. It is preferably equal to or lower than 6.0, preferably equal to or lower than 5.0, preferably equal to or lower than 4.0, preferably lower than 4.0, preferably equal to or lower than 3.5, preferably equal to or lower than 3.0. It is for example equal to about 2.6.

Preferably, the tab-in-tab dosage form according to the disclosure shows linear release of the active ingredient, with a zero-order release kinetics from 0 to 80% dry weight drug release.

Such release can be classically determined by the person skilled in the art by performing a dissolution testing for example using USP dissolution apparatus II equipped with an inline fiber optic system (General test chapter <711> Dissolution - Pharmacopeial Forum Vol.33 No.4 July-Aug. 2007). It can be determined for instance according to the protocol described in the Examples, section 2.1.

Preferably, the floating lag time of the tab-in-tab dosage form according to the disclosure is lower than 18 minutes, preferably equal to or lower than 17 minutes, preferably equal to or lower than 16 minutes, preferably lower than 15 minutes, preferably equal to or lower than 14 minutes, preferably equal to or lower than 13 minutes, preferably equal to or lower than 12 minutes. It is in general equal to or higher than 1 minute, even equal to or higher than 5 minutes, even equal to or higher than 7 minutes, even equal to or higher than 10 minutes.

The floating lag time of the dosage form can be classically determined by the person skilled in the art by performing a dissolution testing for example using USP dissolution apparatus II equipped with an inline fiber optic system (General test chapter <711> Dissolution - Pharmacopeial Forum Vol.33 No.4 July-Aug. 2007). The time required for the tablets to float to the media surface is recorded as floating lag time. This floating lag time can be determined for instance according to the protocol described in the Examples, section 2.2.

Preferably, the gel strength of the tab-in-tab dosage form according to the disclosure is higher than 0.5 N, preferably equal to or higher than 0.7 N, preferably equal to or higher than 0.8 N, preferably equal to or higher than 1.0 N, preferably equal to or higher than 1.2 N, preferably higher than 1.2 N, preferably equal to or higher than 1.5 N, preferably higher than 1.5 N, preferably equal to or higher than 1.8 N, preferably equal to or higher than 2.0 N, preferably higher than 2.0 N, preferably equal to or higher than 2.5 N, preferably equal to or higher than 3 N. It is in general lower than 10.0 N, even equal to or lower than 8.0 N, even equal to or lower than 6.0 N, even equal to or lower than 5.0 N, even equal to or lower than 4.0 N.

The gel strength of the dosage form can be classically determined by the person skilled in the art by performing a dissolution testing for example using USP dissolution apparatus II equipped with an inline fiber optic system (General test chapter <711> Dissolution - Pharmacopeial Forum Vol.33 No.4 July-Aug. 2007). After 8 hours, the hydrated gel strength of the swollen tablets is measured using a texture analyzer. The gel strength can be determined for instance according to the protocol described in the Examples, section 2.3.

Preferably, the tensile strength of the tab-in-tab dosage form according to the disclosure is higher than 1.3 MPa, preferably equal to or higher than 1.5 MPa, preferably equal to or higher than 1.6 MPa, preferably equal to or higher than 1.7 MPa, preferably equal to or higher than 1.8 MPa, preferably equal to or higher than 1.9 MPa, preferably equal to or higher than 2.0 MPa. It is in general equal to or lower than 4.0 MPa, even equal to or lower than 3.5 MPa, even equal to or lower than 3.0 MPa, even equal to or lower than 2.5 MPa.

The tensile strength of the dosage form can be classically determined by the person skilled in the art using the following equation:

$$Tensile\ strenght = \frac{2}{3}\left(\frac{10F}{\pi D^2 \left(2.84\frac{T}{D} - 0.126\frac{T}{W} + 3.15\frac{W}{D} + 0.01\right)}\right)$$

where *F, D, W* and *T* represent the hardness, short axis length, wall height and thickness of tablets respectively.

The tablet tensile strength can be determined for instance according to the protocol described in the Examples, section 2.1.

The invention also covers a process for the manufacture of a floating tab-in-tab dosage form comprising and active ingredient, said process comprising:

- a step of obtaining a first tablet comprising a first portion of said active ingredient; and,
- a step of including said first tablet into a second tablet, said second tablet comprising a second portion of said active ingredient, a release retarding agent, a gelling agent capable of being ionically cross-linked in the presence of divalent cations, an acid-soluble divalent cation and a bicarbonate salt.

This process is particularly useful for preparing tab-in-tab dosage forms according to the disclosure.

Preferably, the first tablet is as defined before for the core of the tab-in-tab dosage form according to the disclosure.

Preferably, the second tablet is as defined before for the shell of the tab-in-tab dosage form according to the disclosure.

Preferably, the step of including the first tablet into the second tablet is performed by filing part of the shell composition into the die, placing the first tablet into said die, covering with the remaining part of the shell composition, and compressing so as to obtain a tab-in-tab dosage form.

Preferably, the first tablet is placed at the center of the die.

Preferably, the weight ratio between the first portion of the shell composition placed into the die and the remaining portion used to cover is of about 1:1.

The instant disclosure also relates to a tab-in-tab dosage form according to the disclosure, or obtainable by the process for the preparation of a tab-in-tab dosage form according to the disclosure, for use as a medicine.

The instant disclosure also relates to the use of the tab-in-tab dosage form according to the disclosure or obtained by the process for the preparation of a tab-in-tab dosage form according to the disclosure, for the preparation of a medicine.

Preferably, the tab-in-tab dosage form is for treating or preventing a disease and/or a condition selected from diabetes, high blood pressure, Parkinson's disease, H. pylori infection, acid reflux.

Typically, the tab-in-tab dosage is for use in a subject in need thereof, preferably for use in humans or animals, preferably for use in mammals, still preferably for use in humans.

The instant disclosure also relates to a method for the treatment or the prevention of a disease and/or of a condition, comprising the administration of the tab-in-tab dosage form according to the disclosure, or obtained by the process for the preparation of a tab-in-tab dosage form according to the disclosure, to a subject in need thereof. Preferably, the disease and/or condition is as described before. Preferably, the subject in need thereof is as described before.

In the instant disclosure, the amounts of ingredients are generally expressed in percentages by weight. Unless otherwise specified these weights are amounts of ingredients as such, in their powdery or oily form. Powdery ingredients generally include small amount of water (also referred as %moisture or as "loss on drying") and eventually small amounts of impurities.

Other characteristics and advantages of the present invention will emerge clearly on reading the examples given hereinafter, which illustrate the invention without however limiting it.

## Examples

### 1. Tablets' preparation

The inventors prepared tablets having different formula as shown in Figure 1. Different tab-in-tab dosage forms (comparative or according to the invention) were prepared (herein after referred as "Ttab-X"). Comparative monolithic dosage forms were also prepared (herein after referred as "mono-X").

As an active ingredient (API), metformin was used.

As release retarding agent, a co-processed HPMC / mannitol compound according to patent US 9,234,049 B2 having a weight ratio HPMC K4M : mannitol of 70:30 was used (HPMC-man).

As a gelling agent capable of being ionically cross-linked in the presence of divalent cations, sodium alginate (NaAlg) was used.

As an acid-soluble divalent cation, calcium carbonate ($CaCO_3$) was used. As a carbonate, sodium bicarbonate (NaHCOs) was used.

Formulations used are presented in Figure 1.

All materials were de-lumped using a 710 μm aperture size sieve, pre-mixed geometrically using a spatula and subsequently mixed using a tumble blender (Turbula®, WAB, Switzerland) at 42 rpm for 10 min. Tablets were compacted using a compaction simulator (Styl'One, MedelPharm, France) at a compression speed of 40 mm/s. The tools were externally lubricated with magnesium stearate for 500 ms at 3 bars.

Monolithic matrix tablets were of 1000 mg and were compressed with an elliptical concave punch set (Natoli Engineering Company, USA). The long axis of the punch set was 17 mm while the short axis of the punch set was 9 mm. Compression force was varied to achieve a target porosity of 15 %.

The tab-in-tab dosage forms were prepared as follow: for the core, tablets of 100-170 mg were compacted using the compaction simulator, equipped with a 5 mm concave punch set (Natoli Engineering Company, USA), and compressed to a target tensile strength of 1 MPa. Tablets of 900, 1000 or 1100 mg were compressed using an elliptical concave punch set (Natoli Engineering Company, USA). The long axis of the punch set was 17 mm while the short axis of the punch set was 9 mm. Half of the shell formulation was first filled into the die. Subsequently, the tablet cores were manually placed in the center of the powder bed within the die. A tamping force of 1 kN was applied before the die was filled with the remaining shell formulation and subsequently compressed at a pre-determined force to produce compacts with a target porosity of 15 %.

## 2. Tablets' evaluation

### 2.1. Release profile (dissolution study) and tensile strength

The monolithic and tab-in-tab dosage forms were first characterized and compared for their API release profile and tensile strength.

Dissolution testing was conducted using USP dissolution apparatus II (708-DS, Agilent, USA) equipped with an inline fibre optic system. Pre-weighed tablets (W1) were added to 900 mL of simulated gastric fluid degassed and warmed by vacuum filtration (EzFill 4500, Distek, USA). The paddle speed was set to 50 rpm and the temperature was maintained at 37±0.5°C. Inline measurements were taken and analysed with a UV spectrophotometer (Cary 60, Agilent, USA) at a wavelength of 232 nm and 400 nm. Each experiment was conducted in triplicates.

Tablet dimensions were measured using a digital micrometer screw gauge (Mitutoyo, Japan) and tablet hardness determined using a hardness tester (TBH 425, Erweka, Germany). Tablet properties were only determined 24 h after compaction. Tensile strength (K.G. Pitt and M.G. Heasley, Determination of the tensile strength of elongated tablets. Powder Technology, 2013. 238: p. 169-175) of at least 6 tablets per run were calculated using equation Math.1.

[Math.1]

$$Tensile\ strenght = \frac{2}{3}\left(\frac{10F}{\pi D^2 \left(2.84\frac{T}{D} - 0.126\frac{T}{W} + 3.15\frac{W}{D} + 0.01\right)}\right)$$

where F, D, W and T represent the hardness, short axis length, wall height and thickness of tablets respectively.
The results are presented in Figures 2 and 3 as well as in Table 1.

[Table 1]

|  | Zero order release |
|---|---|
| **Mono-1** | Fail |
| **Mono-2** | Fail |
| **Mono-3** | Fail |
| **Mono-4** | Fail |
| **Mono-5** | Fail |
| **Mono-6** | Fail |
| **Ttab-1** | Pass |
| **Ttab-2** | Pass |
| **Ttab-3** | Pass |
| **Ttab-4** | Pass |
| **Ttab-5** | Pass |
| **Ttab-6** | Pass |
| **Ttab-7** | Pass |
| **Ttab-8** | Fail |

(continued)

|  | Zero order release |
| --- | --- |
| Ttab-9 | Pass |
| Ttab-10 | Pass |

Monolithic dosage forms (Mono-1 to -6) show weak tensile strength, below 1.5 MPa, even when high compression force was employed. Based on visual observations, monolithic tablets eroded rapidly when immersed in dissolution media (simulated gastric fluid). On the contrary, tab-in-tab dosage forms (Ttab-1 to -10) exhibited a high tensile strength (Figure 2).

In addition, zero order release kinetics was observed ($R^2$ = 0.99) with all tab-in-tab dosage forms according to the invention Ttab-1 to -7, and also with comparative Ttab-9 and Ttab-10. Zero order release could not be obtained with monolithic matrix tablets, or when removing the bicarbonate salt from the tab-in-tab formulation (Ttab-8) (Figure 3).

These results show that the tab-in-tab dosage forms according to the disclosure allow to obtain linear release of the drug and good tensile strength, in a broad range of formula.

### 2.2. Floating lag time

Tab-in-tab dosage forms were then evaluated for their floating lag time.

The floating behavior of tablets was examined using similar conditions as described in dissolution studies: tablets were added to 900 mL of degassed simulated gastric fluid at 37±0.5°C and agitated at 50 rpm using USP dissolution apparatus II. The time required for the tablets to float to the media surface was recorded as floating lag time. The total time where the tablet was floating on the media was recorded as floating duration. Each measurement represented a mean of at least three replicates.

Results are presented in Figures 4.

The average floating lag time of the tab-in-tab dosage forms according to the invention Ttab-1 to -7 is good as it is always lower than 15 minutes. Comparative Ttab-9 (free of divalent cation) showed good floating lag time. However, as it is apparent from the next results (2.3.), this formula does not allow to achieve satisfying gel strength of the dosage form. As for comparative dosage forms Ttab-8 (free of bicarbonate) and Ttab-10 (free of gelling agent, divalent cation and bicarbonate) they exhibited too high floating lag-time to be efficient. Furthermore, it is reminded that zero-order release of the drug could not be obtained with Ttab-8. Without being bound by any theory, it is believed that the bicarbonate functions as a gas generating agent and pH modifier. It is believed that it shortens the floating lag time due to its high reactivity. At the same time, the bicarbonate provides the microenvironment pH for dissolution of the gelling agent (e.g. alginate). Without it, the gelling agent is in its acid form (e.g. alginic acid) in acidic gastric fluid, which is insoluble and has disintegrant properties. As a result, zero order release cannot be achieved and the resultant gel is very weak (see the next results on gel strength).

The best results (floating lag time consistently below 15 minutes) were obtained with Ttab-5 and Ttab-7, having a release retarding agent / gelling agent weight ratio greater than 1.5 and lower than 3.0 in the shell, an also with Ttab-1, having a greater portion of API in the shell.

### 2.3. gel strength

Tab-in-tab dosage forms according to the invention (Ttab-5 to -7) and comparative tab-in-tab dosage forms (Ttab-8 to -10) were then evaluated for their gel strength.

Gel strength was evaluated using the same conditions as described in dissolution studies. Tablets were added to 900 mL of degassed simulated gastric fluid at 37±0.5°C and agitated at 50 rpm using USP dissolution apparatus II. The method for gel strength studies was adapted from Kim et al. (H. Kim et al., Mechanically robust gastroretentive drug-delivery systems capable of controlling dissolution behaviors of coground β-lapachone. Pharmaceutics, 2019. 11(6): p. 271). After 8h, the hydrated gel strength of the swollen tablets was measured using a texture analyser (TA.XTplus, Stable Micro System, UK), equipped with a 5 kg load cell and exponent software. Force recording was initiated when trigger force reached 0.001 N. The 20 mm round flat-faced aluminium probe compressed the swollen matrix at a rate of 2 mm/s to mimic the compression rate in the stomach (B. Laulicht et al., Understanding gastric forces calculated from high-resolution pill tracking. Proceedings of the National Academy of Sciences, 2010. 107(18): p. 8201-8206) until 50 % of the initial thickness and the maximum force at the 50 % strain represented the gel strength. Each measurement represented a mean of at least triplicates.

The results are presented in Figure 5.

The gel strength of the tab-in-tab dosage forms according to the invention Ttab-1 to -7 is higher, as compared to the gel strength of the comparative tab-in-tab dosage forms Ttab-8 and Ttab-9. We can conclude that both the bicarbonate and the divalent cation are required to obtain a better gel strength. Comparative Ttab-10 exhibits a better gel strength, but it is reminded that this tablet doesn't float (section 2.2.) such that it cannot be used as a floating tablet.

Results obtained with Ttab-5 to -7 are excellent, as the gel strength is ideally higher than 2N. That is to say that excellent gel strength could be obtained when using a release retarding agent / gelling agent weight ratio below 3.0 in the shell.

### 2.4. Conclusion

Taking all this together, the tab-in-tab dosage forms according to the disclosure allow: to obtain a linear release of a drug, while exhibiting satisfying tensile strength and floating lag time. Furthermore, good gel strength could be obtained, in particular when using a release retarding agent / gelling agent weight ratio below 3.0 in the shell. Even better results could be obtained when using a release retarding agent / gelling agent weight ratio greater than 1.5 in the shell. Excellent results were obtained with Ttab-5 and -7: they can achieve zero-order release of the drug, they have good tensile strength, a floating lag time consistently below 15 minutes, and a gel strength greater than 2N.

### Claims

1. A floating tab-in-tab dosage form comprising an active ingredient, said tab-in-tab dosage form comprising a core (a) and a shell (b), wherein:

   the core (a) comprises a first portion of said active ingredient;
   the shell (b) comprises:

      - a second portion of said active ingredient; and,
      - a release retarding agent; and,
      - a gelling agent capable of being ionically cross-linked in the presence of divalent cations; and,
      - an acid-soluble divalent cation; and,
      - a bicarbonate salt.

2. The floating tab-in-tab dosage form of claim 1, wherein said acid-soluble divalent cation is selected from carbonate salts, preferably from calcium carbonate, magnesium carbonate, zinc carbonate, calcium chloride, or from a mixture thereof.

3. The floating tab-in-tab dosage form of claim 1 or 2, wherein said bicarbonate salt is selected from ammonium bicarbonate, lithium bicarbonate, potassium bicarbonate, sodium bicarbonate, or from a mixture thereof.

4. The floating tab-in-tab dosage form according to any of claims 1 to 3, wherein said release retarding agent is selected from hydroxypropyl methylcellulose (HPMC), hydroxyethylcellulose (HEC), hydroxypropyl cellulose (HPC), native or chemically modified pregelatinized starches, guar gum, gellan gum, xanthan gum, their derivatives, or from a mixture thereof.

5. The floating tab-in-tab dosage form according to any of claims 1 to 4, wherein said gelling agent capable of being ionically cross-linked in the presence of divalent cations is selected from alginate, pectinate, iota-carrageenan, gellan gum, carboxymethylcellulose, or from a mixture thereof.

6. The floating tab-in-tab dosage form according to any of claims 1 to 5, wherein the weight ratio of release retarding agent / gelling agent of the shell is equal to or higher than 0.5, preferably equal to or higher than 1.0, preferably equal to or higher than 1.5, preferably higher than 1.5, preferably equal to or higher than 2.0.

7. The floating tab-in-tab dosage form according to any of claims 1 to 6, wherein the weight ratio of release retarding agent / gelling agent of the shell is equal to or lower than 5.0, preferably equal to or lower than 4.5, preferably equal to or lower than 3.5, preferably equal to or lower than 3.0, more preferably lower than 3.0, preferably equal to or lower than 2.5.

8. The floating tab-in-tab dosage form according to any of claims 1 to 7, wherein the amount of said active ingredient is equal to or higher than 10% by weight with respect to the total weight of the tab-in-tab dosage form, preferably equal to or higher than 20%, preferably equal to or higher than 30%, preferably equal to or higher than 40%, preferably equal to or higher than 45%.

9. The floating tab-in-tab dosage form according to any of claims 1 to 8, wherein the weight ratio between the first and second portion of said active ingredient is equal to or higher than 0.2, preferably equal to or higher than 0.4, preferably equal to or higher than 0.6, preferably equal to or higher than 0.7.

10. The floating tab-in-tab dosage form according to any of claims 1 to 9, wherein the weight ratio between the first and second portion of said active ingredient is equal to or lower than 3.0, preferably equal to or lower than 2.5, preferably equal to or lower than 2.0, preferably equal to or lower than 1.7, preferably equal to or lower than 1.5, more preferably lower than 1.5, preferably equal to or lower than 1.4, preferably equal to or lower than 1.3, preferably equal to or lower than 1.2, preferably equal to or lower than 1.1.

11. A process for the manufacture of a floating tab-in-tab dosage form comprising an active ingredient, said process comprising:

   - a step of obtaining a first tablet comprising a first portion of said active ingredient; and,
   - a step of including said first tablet into a second tablet, said second tablet comprising a second portion of said active ingredient, a release retarding agent, a gelling agent capable of being ionically cross-linked in the presence of divalent cations, an acid-soluble divalent cation and a bicarbonate salt.

12. A floating tab-in-tab dosage form as defined in any of claims 1 to 10, or obtainable by the process any of claim 11, for use as a medicine.

[Fig. 1]

| Trial ref. | Total composition | | | | | Shell composition | | | | | | Core composition | | Shell / core ratio |
|---|---|---|---|---|---|---|---|---|---|---|---|---|---|---|
| | API | HPMC-man (HPMC) | NaAlg | CaCO₃ | NaHCO₃ | API | HPMC-man (HPMC) | NaAlg | CaCO₃ | NaHCO₃ | HPMC / NaAlg ratio | API | HPMC-man (HPMC) | |
| Mono-1 | 50 | 29 (21) | 7 | 7 | 7 | NA | NA | NA | NA | NA | 2.9* | NA | NA | NA |
| Mono-2 | 50 | 35 (25) | 5 | 5 | 5 | NA | NA | NA | NA | NA | 4.9* | NA | NA | NA |
| Mono-3 | 50 | 41 (39) | 3 | 3 | 3 | NA | NA | NA | NA | NA | 9.6* | NA | NA | NA |
| Mono-4 | 50 | 33 (23) | 7 | 5 | 5 | NA | NA | NA | NA | NA | 3.3* | NA | NA | NA |
| Mono-5 | 50 | 31 (22) | 9 | 5 | 5 | NA | NA | NA | NA | NA | 2.4* | NA | NA | NA |
| Mono-6 | 50 | 28 (20) | 12 | 5 | 5 | NA | NA | NA | NA | NA | 1.6* | NA | NA | NA |
| Ttab-1 | 50 | 35 (25) | 5 | 5 | 5 | 32 | 33 (23) | 5 | 5 | 5 | 4.6 | 18 | 2 (1.4) | 4.0 |
| Ttab-2 | 50 | 35 (25) | 5 | 5 | 5 | 20 | 31.5 (22) | 5 | 5 | 5 | 4.4 | 30 | 3.5 (2.5) | 1.9 |
| Ttab-3 | 50 | 35 (25) | 5 | 5 | 5 | 25 | 32 (22) | 5 | 5 | 5 | 4.5 | 25 | 3 (2.1) | 2.6 |
| Ttab-4 | 50 | 33 (23) | 7 | 5 | 5 | 25 | 30 (21) | 7 | 5 | 5 | 3.0 | 25 | 3 (2.1) | 2.6 |
| Ttab-5 | 50 | 31 (22) | 9 | 5 | 5 | 25 | 28 (20) | 9 | 5 | 5 | 2.2 | 25 | 3 (2.1) | 2.6 |
| Ttab-6 | 50 | 28 (20) | 12 | 5 | 5 | 25 | 25 (18) | 12 | 5 | 5 | 1.5 | 25 | 3 (2.1) | 2.6 |
| Ttab-7 | 55 | 31 (22) | 9 | 3 | 3 | 30 | 28 (20) | 9 | 3 | 3 | 2.2 | 25 | 3 (2.1) | 2.6 |
| Ttab-8 | 50 | 31 (22) | 9 | 10 | 0 | 25 | 28 (20) | 9 | 10 | 0 | 2.2 | 25 | 3 (2.1) | 2.6 |
| Ttab-9 | 50 | 31 (22) | 9 | 0 | 10 | 25 | 28 (20) | 9 | 0 | 10 | 0 | 25 | 3 (2.1) | 2.6 |
| Ttab-10 | 50 | 50 (35) | 0 | 0 | 0 | 25 | 47 (33) | 0 | 0 | 0 | NA | 25 | 3 (2.1) | 2.6 |

Amounts of ingredients are expressed in %, w/w.

*ratio in the total tablet.

NA: not applicable

[Fig. 2]

[Fig. 3]

Cumulative drug release (%) — Mono-1, Mono-2, Mono-3, Mono-4, Mono-5, Mono-6 versus Time (hours)

Cumulative drug release (%) — Ttab-1, Ttab-2, Ttab-3, Ttab-4, Ttab-5, Ttab-6, Ttab-7, Ttab-8, Ttab-9, Ttab-10 versus Time (hours)

[Fig. 4]

Floating lag time (min)

[Fig. 5]

Gel strength at 8h (N)

Europäisches
Patentamt

European
Patent Office

Office européen
des brevets

**EUROPEAN SEARCH REPORT**

Application Number

EP 22 30 6124

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | QI XIAOLE ET AL: "Floating tablets for controlled release of ofloxacin via compression coating of hydroxypropyl cellulose combined with effervescent agent", INTERNATIONAL JOURNAL OF PHARMACEUTICS, ELSEVIER, NL, vol. 489, no. 1, 5 May 2015 (2015-05-05), pages 210-217, XP029183108, ISSN: 0378-5173, DOI: 10.1016/J.IJPHARM.2015.05.007 * the whole document * * table 1 * | 1-12 | INV. A61K9/24 |
| Y,D | CN 104 688 703 A (UNIV CHINA PHARMA) 10 June 2015 (2015-06-10) * examples * | 1-12 | |
| Y | WO 2005/020879 A2 (VECTA LTD [IL]; DAVID AYELET [IL]; GLOZMAN SABINA [IL]; PAUL LADA [IL]) 10 March 2005 (2005-03-10) * page 12, line 32 – page 13, line 2 * | 1-12 | |
| | | | TECHNICAL FIELDS SEARCHED (IPC) |
| A | RAZA ALI ET AL: "Formulation of zein based compression coated floating tablets for enhanced gastric retention and tunable drug release", EUROPEAN JOURNAL OF PHARMACEUTICAL SCIENCES, vol. 132, 26 January 2019 (2019-01-26), pages 163-173, XP085644571, ISSN: 0928-0987, DOI: 10.1016/J.EJPS.2019.01.025 * table 1 * * figures * | 1-12 | A61K |

-/--

The present search report has been drawn up for all claims

| Place of search | Date of completion of the search | Examiner |
|---|---|---|
| Munich | 5 January 2023 | S. von Eggelkraut-G. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 22 30 6124

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | DAS SUPRATIM ET AL: "Gastro-retentive drug delivery systems: a recent update on clinical pertinence and drug delivery", DRUG DELIVERY AND TRANSLATIONAL RESEARCH, SPRINGER, GERMANY, vol. 11, no. 5, 5 January 2021 (2021-01-05), pages 1849-1877, XP037558053, ISSN: 2190-393X, DOI: 10.1007/S13346-020-00875-5 [retrieved on 2021-01-05] * the whole document * | 1-12 | |

TECHNICAL FIELDS
SEARCHED (IPC)

The present search report has been drawn up for all claims

| Place of search | Date of completion of the search | Examiner |
|---|---|---|
| Munich | 5 January 2023 | S. von Eggelkraut-G. |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

& : member of the same patent family, corresponding
   document

EPO FORM 1503 03.82 (P04C01)

**ANNEX TO THE EUROPEAN SEARCH REPORT
ON EUROPEAN PATENT APPLICATION NO.**

EP 22 30 6124

This annex lists the patent family members relating to the patent documents cited in the above-mentioned European search report.
The members are as contained in the European Patent Office EDP file on
The European Patent Office is in no way liable for these particulars which are merely given for the purpose of information.

05-01-2023

| Patent document cited in search report | | Publication date | Patent family member(s) | | Publication date |
|---|---|---|---|---|---|
| CN 104688703 | A | 10-06-2015 | NONE | | |
| WO 2005020879 | A2 | 10-03-2005 | AU | 2004268446 A1 | 10-03-2005 |
| | | | CA | 2536906 A1 | 10-03-2005 |
| | | | EP | 1658089 A2 | 24-05-2006 |
| | | | JP | 2007503427 A | 22-02-2007 |
| | | | KR | 20060083198 A | 20-07-2006 |
| | | | WO | 2005020879 A2 | 10-03-2005 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 104688703 A
- US 9234049 B2

### Non-patent literature cited in the description

- Dissolution - Pharmacopeial Forum. July 2007, vol. 33
- **K.G. PITT ; M.G. HEASLEY.** Determination of the tensile strength of elongated tablets. *Powder Technology,* 2013, vol. 238, 169-175
- **H. KIM et al.** echanically robust gastroretentive drug-delivery systems capable of controlling dissolution behaviors of coground β-lapachone. *Pharmaceutics,* 2019, vol. 11 (6), 271
- **B. LAULICHT et al.** Understanding gastric forces calculated from high-resolution pill tracking. *Proceedings of the National Academy of Sciences,* 2010, vol. 107 (18), 8201-8206